# FASCICULE DE BREVET EUROPEEN

(11) **EP 2 175 806 B1**
(45) Date de publication et mention de la délivrance du brevet: **24.09.2014**
(21) Numéro de dépôt: 08826543.4
(22) Date de dépôt: 10.07.2008
(51) Int. Cl.: A61F 2/16

(54) **IMPLANT INTRAOCULAIRE SOUPLE A HAPTIQUE CIRCULAIRE**
FLEXIBLES INTRAOKULARIMPLANTAT MIT ZIRKULÄRER HAPTIK
FLEXIBLE INTRAOCULAR IMPLANT WITH CIRCULAR HAPTIC

(30) Priorité: 13.07.2007 FR 0756474
(43) Date de publication de la demande: 21.04.2010
(73) Titulaire: Sourdille, Philippe, 86800 Liniers (FR)
(72) Inventeur: PRIVAT DE FORTUNE, Matthieu, F-74960 Cran Gevrier (FR); JORON, Yannick, F-74370 Pringy (FR); GANTIN, Denis, F-74130 Ayze (FR)
(74) Mandataire: Cabinet Plasseraud
(86) Numéro de dépôt international: PCT/FR2008/051301
(87) Numéro de publication internationale: WO 2009/013421

(56) Documents cités:
- EP-A- 0 592 813
- WO-A1-2007/079315
- DE-A1- 3 722 910
- FR-A- 2 786 686
- FR-A- 2 841 122

## Description

La présente invention a pour objet un implant intraoculaire souple à haptique circulaire.

Comme cela est bien connu, deux types de matériaux peuvent être utilisés pour réaliser des implants intraoculaires :
soit des matériaux dits rigides tels que le PMMA ; soit des matériaux dits souples tels que des matériaux acryliques hydrophiles ou hydrophobes dont un exemple est connu sous la marque Hydrogel. La présente invention concerne un implant réalisé avec ce deuxième type de matériau.

Il est également connu que les implants intraoculaires sont constitués par deux parties essentielles qui sont d'une part, une partie optique constituant le système correcteur de la vision de l'implant et d'autre part, une partie haptique qui sert à maintenir la partie optique centrée dans l'oeil et, dans le cas particulier considérer dans le sac capsulaire de l'oeil après ablation du cristallin.

La partie haptique de l'implant intraoculaire peut prendre différentes formes dont les principaux types sont des anses haptiques en forme de C ou de J ou encore une forme circulaire reliée à la partie optique par des bras. L'implant intraoculaire objet de l'invention concerne ce dernier type de partie haptique.

L'implant intraoculaire, une fois mis en place dans l'oeil, doit bien sûr réaliser la correction optique souhaitée pour le patient. Cette correction dépend non seulement des caractéristiques de la partie optique mais également de la position de l'implant intraoculaire à l'intérieur de l'oeil.

Il est donc important que l'implant conserve la position dans l'oeil prévue initialement par le chirurgien. Or, il s'avère que, après la mise en place de l'implant dans le sac capsulaire, celui-ci a tendance à diminuer de diamètre dans la période qui suit la mise en place de l'implant. Cette réduction de diamètre peut atteindre 10 %.

On comprend, que lors de la réduction du diamètre du sac capsulaire, avec des implants de type classique, il y a lieu de craindre que les contraintes appliquées par le sac capsulaire sur la partie haptique du fait de sa réduction de diamètre n'entraînent un déplacement axial de la partie optique selon la direction de l'axe optique de l'oeil. De plus, cette réduction de diamètre peut provoquer des contraintes qui vont entraîner une déformation non de révolution circulaire de la partie haptique, ce qui peut entraîner une prise d'angulation de l'axe optique de l'implant intraoculaire par rapport à l'axe optique de l'oeil du patient. Ces deux risques peuvent bien sûr entraîner une gêne très sensible pour le patient ayant reçu l'implant intraoculaire.

Le document FR 2 841 122 décrit un implant pouvant présenter les risques évoqués ci-dessus.

De plus, le document DE 37 22910 décrit une lentille intraoculaire qui comprend une partie haptique faite de deux boucles raccordées à la périphérie de la partie optique. Le document FR 2 786 686 décrit un implant intraoculaire en matériau souple comprenant une partie haptique de forme annulaire fermée. Le document EP 592 813 décrit aussi des implants intraoculaires.

Un objet de la présente invention est donc de fournir un implant intraoculaire réalisé en un matériau souple qui comporte une partie haptique de forme générale circulaire qui se déforme de telle manière que sous l'effet de la réduction du diamètre du sac capsulaire, elle n'entraîne aucun déplacement sensible de la partie optique selon la direction de son axe optique et aucune prise d'angulation significative de l'axe optique de l'implant par rapport à l'axe optique de l'oeil.

Pour atteindre ce but, selon l'invention, l'implant intraoculaire souple destiné à être placé dans un sac capsulaire, comprend une partie optique de forme sensiblement circulaire et présentant un axe optique et une partie haptique raccordée à la périphérie de la partie optique, et il se caractérise en ce que ladite partie haptique comprend :
- une partie de contact constituée par n éléments de contact (n>2) en forme d'arcs de cercle ayant tous le même rayon de courbure et dont le bord externe est au contact de la zone équatoriale du sac capsulaire et n éléments de raccordement, chaque élément de raccordement étant raccordé à une extrémité de deux éléments de contact consécutifs, ayant une forme incurvée déformable, et présentant une rigidité très inférieure à celle des éléments de contact ; et
- n bras de raccordement déformables ayant chacun une première extrémité raccordée à la périphérie de la partie optique et une deuxième extrémité raccordée à un élément de contact, par quoi, sous l'effet de la diminution du diamètre du sac capsulaire, les éléments de raccordement et les bras de raccordement se déforment sans entraîner de déplacement significatif de la partie optique selon la direction de son axe optique, et sans introduire de prise d'angulation significative de l'axe optique de l'implant par rapport à l'axe optique de l'oeil du patient.

On comprend que, grâce à la différence de rigidité des parties de contact et des éléments de raccordement, ce sont ces derniers qui sous l'effet des contraintes appliquées par la réduction du diamètre du sac capsulaire vont se déformer réduisant ainsi le diamètre externe de la partie haptique de l'implant constituée par les éléments de contact. Sous l'effet de cette réduction de diamètre, les bras de raccordement se déforment également. Ainsi, la partie optique de l'implant intraoculaire ne subit pas de déplacement significatif selon la direction de son axe optique et, compte tenu de la symétrie de révolution de la partie haptique, cette déformation est régulière et de révolution et n'entraîne donc pas une prise d'angulation de l'axe optique de la partie optique par rapport à l'axe optique de l'oeil.

De préférence, l'implant intraoculaire est caractérisé en ce que les bras de raccordement et les éléments de raccordement présentent une section droite très inférieure à la section droite des éléments de contact.

De préférence également, l'implant intraoculaire est caractérisé en ce que chaque élément de contact comporte un bord sensiblement parallèle à l'axe optique et deux faces respectivement antérieure et postérieure, le bord faisant avec au moins une desdites faces un angle inférieur à 120 degrés formant ainsi une arête ou bord carré apte à exercer une pression localisée importante sur ledit sac capsulaire.

On comprend que grâce à la présence de ce bord carré ou d'arête vive qui existe sur les éléments de contact de l'haptique, celles-ci exercent une pression localisée sur le sac capsulaire. Cette pression localisée permet d'éviter la prolifération de cellules pathogènes qui se développent souvent dans la zone équatoriale du sac capsulaire et qui ont tendance à migrer vers la paroi postérieure du sac capsulaire. On évite ainsi l'apparition d'une cataracte secondaire par opacification de la partie postérieure du sac capsulaire.

De préférence également, les éléments de contact et les éléments de raccordement forment une pièce annulaire fermée.

D'autres caractéristiques et avantages de l'invention apparaîtront mieux à la lecture de la description qui suit d'un mode de réalisation préféré de l'invention donné à titre d'exemples non limitatifs. La description se réfère aux figures annexées, sur lesquelles :
- la figure 1 est une vue de face de l'implant intraoculaire au repos, c'est-à-dire sans application de contrainte sur la partie haptique ;
- la figure 2 est une vue de côté de l'implant intraoculaire ; et
- la figure 3 est une vue de face partielle montrant la partie haptique de l'implant déformé ; et
- la figure 4 est une vue simplifiée de côté montrant la mise en place de l'implant intraoculaire dans le sac capsulaire.

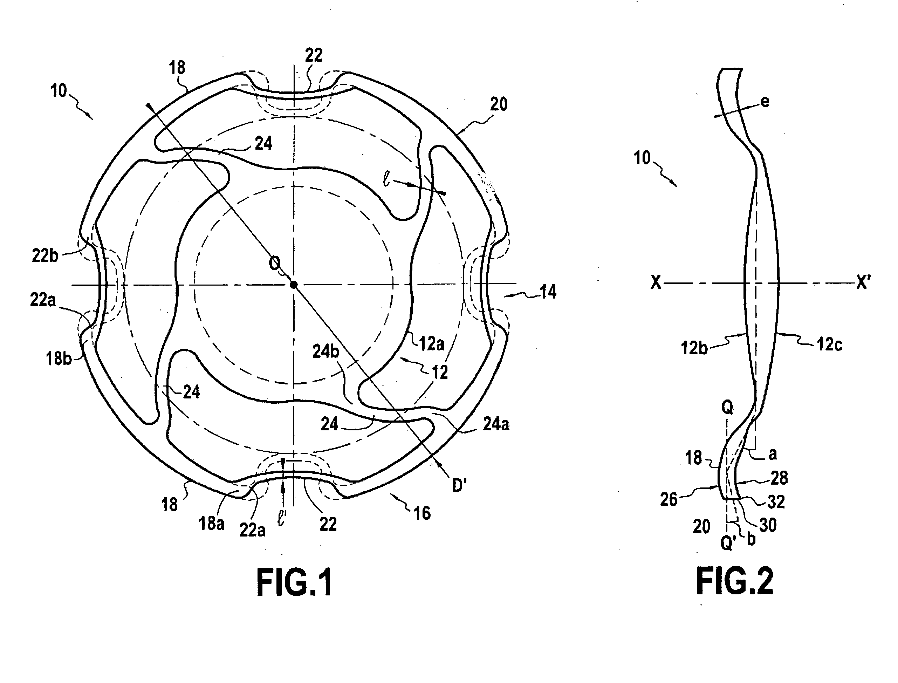

En se référant tout d'abord aux figures 1 et 2, on va décrire les principaux éléments constitutifs de l'implant intraoculaire 10 selon un mode préféré de mise en oeuvre de l'invention.

L'implant intraoculaire 10 est réalisé à partir d'un matériau dit souple typiquement un acrylique hydrophile ou hydrophobe dont un exemple particulier est le produit commercialisé sous la marque Hydrogel. De préférence, l'implant est monobloc.

L'implant 10 est constitué par une partie optique 12 de forme générale circulaire présentant un bord 12a, un dioptre antérieur 12b et un dioptre postérieur 12c. L'implant intraoculaire comporte également une partie haptique 14 constituée par un anneau circulaire externe 16 constitué par n éléments de contact 18 ayant un bord externe 20 et n éléments de raccordement 22 de forme incurvée interposés entre deux éléments de contact 18 consécutifs. Dans l'exemple considéré, n est égal à 4 ce qui correspond à une solution optimale. Cependant bien entendu n pourrait être différent mais en tout cas au moins égal à 2.

De préférence, tous les éléments ce contact 18 correspondent à une même longueur d'arc circulaire. Il en va de même pour les éléments de raccordement 22. Grâce à la liaison qui existe entre les extrémités des éléments de contact et les éléments de raccordement, ces deux types d'élément forment une pièce unique, sans interruption, constituant ainsi un anneau sensiblement circulaire fermé.

L'anneau 16 constituant l'essentiel de la partie haptique est raccordé à la périphérie 12a de la partie optique 12 par n bras 24. Dans l'exemple considéré, il y a quatre bras de raccordement. Chaque élément de contact 18 a la forme d'un arc de cercle au repos centré sur le centre optique O de la partie optique 12 ou plus précisément sur son axe optique X,X'. Les extrémités 18a et 18b de chaque élément de contact 18 sont raccordés aux extrémités 22a ou 22b d'un élément de raccordement 22 adjacent. Chaque bras 24 comporte une première extrémité 24a de raccordement à un élément de contact 18 dans sa partie centrale et une deuxième extrémité 24b de raccordement à la périphérie de la partie optique 12, ces extrémités comportant des congés assurant une certaine rigidification au niveau des deux raccordements.

Les éléments de contact 18 ont une section droite telle qu'ils soient beaucoup plus rigides que les éléments de raccordement 22 du moins dans un plan parallèle au plan optique de la partie optique 12 de l'implant intraoculaire. Typiquement, l'épaisseur e des bras 24 (selon la direction de l'axe X,X') est très supérieure, au moins égale à deux fois, la largeur l de ces mêmes bras. De la même manière, la largeur l' de chaque élément de raccordement 22 est très inférieure à son épaisseur e'.

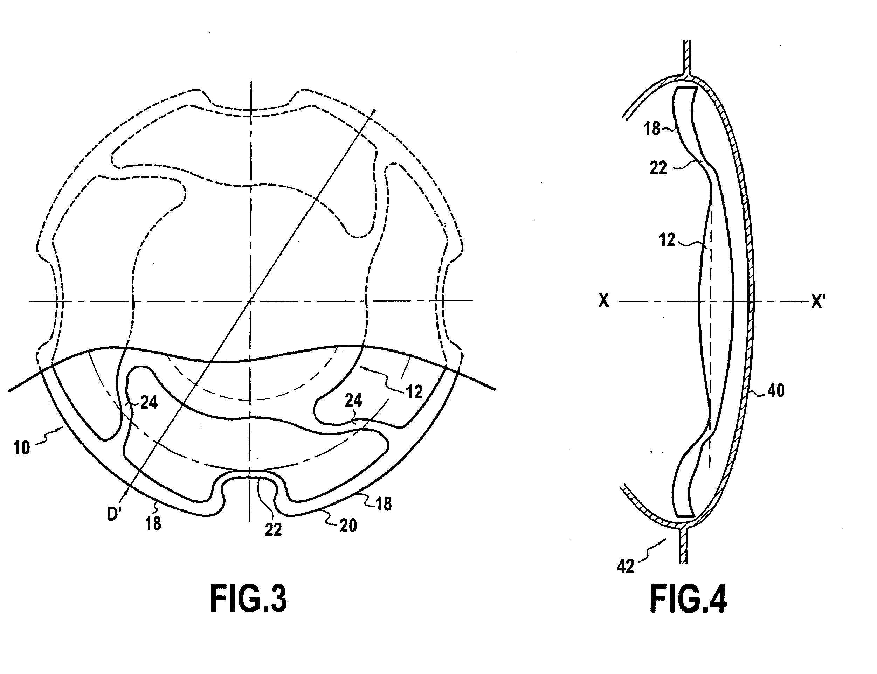

Comme on le voit mieux sur la figure 2, les bras 24 présentent une angulation a vers l'avant c'est-à-dire vers le dioptre antérieur 12b par rapport au plan optique PP' de la partie optique 12. En revanche, les éléments de contact 18 présentent une angulation b vers l'arrière par rapport à un plan QQ' parallèle au plan optique PP'. La première angulation a des bras par rapport au plan optique assure le maintien du dioptre postérieur 12c de la partie optique 12 contre la partie postérieure 40 du sac capsulaire 42 comme le montre la figure 4. L'angulation b des éléments de contact 18 assure une poussée de la partie optique vers l'avant qui maintient de façon stable la partie optique 12 contre la paroi postérieure 40 du sac capsulaire ainsi que cela a été décrit.

Comme le montre mieux la figure 2, les éléments de contact 18 comportent une face antérieure 26, une face postérieure 28 et un rebord 30. Les faces postérieures 28 et le rebord 30 sont déterminés de telle manière qu'ils constituent au moins une arête ou bord carré 32 apte à exercer une pression locale significative sur le sac capsulaire dans la région équatoriale pour former ce qui est appelé le plus souvent un "bord carré" comme on l'a déjà expliqué ce bord carré est apte à bloquer la prolifération de cellules pathogènes de la zone équatoriale du sac capsulaire vers sa paroi postérieure 40.

L'angle entre la face postérieure 28 et le rebord 30 est inférieur à 120 degrés pour constituer l'arête. Dans l'exemple représenté, cet angle est inférieur à 90 degrés en raison de l'angulation b des éléments de contact 18.

Ainsi qu'on l'a déjà expliqué, les éléments de raccordement 22 ont dans un plan parallèle au plan optique une rigidité très inférieure à celle des éléments de contact 18. Ainsi sous l'effet de la contraction déjà mentionnée du sac capsulaire qui produit donc une diminution du diamètre de celui-ci et grâce au fait que l'ensemble des éléments de contact et des éléments de raccordement forme un anneau fermé, les contraintes résultant de cette contraction appliquées aux bords externes des éléments de contact 18 vont provoquer un fléchissement des éléments de raccordement 22 sans modification de la forme des éléments de contact 18 de telle manière que le diamètre externe D' défini par le bord externe des éléments de contact 18 devienne égal au diamètre réduit du sac capsulaire. Cette déformation localisée dans les zones de raccordement 22 évite d'exercer une poussée vers l'arrière sur la partie optique 12 puisqu'en outre, les bras 24 présentent une flexibilité relativement importante dans des directions sensiblement orthogonales à l'axe optique XX' de la partie optique 12. La déformation des bras 24 suit naturellement la réduction du diamètre externe D' défini par les bords externes des éléments de contact 18.

Du fait qu'en a, à la fois, réduction du diamètre du cercle sur lequel sont disposés les bords extérieurs 20 des éléments de contact 18, et diminution de la distance entre les extrémités 24a et 24b des bras 24, il n'y a sensiblement pas de déplacement.

Dans l'exemple particulier décrit, le diamètre externe D de l'implant intraoculaire au repos est égal à 10,5 mm. L'anneau 16 peut se contracter de telle manière qu'on obtienne un diamètre externe de l'haptique contractée D' de l'ordre de 8 mm sans déplacement significatif de la partie optique 12 selon la direction de l'axe optique de l'implant XX'. En outre, compte tenu de la symétrie de révolution de l'anneau 16, les diminutions de diamètre par déformation localisée de cet anneau sont régulières. En conséquence, cette diminution de diamètre n'entraîne aucune prise d'angulation significative de l'axe optique XX' de la partie optique 12 par rapport à l'axe optique naturel de l'oeil XX'.

D'autres modes de réalisation pourraient être envisagés dès lors que la partie haptique est constituée essentiellement par un anneau au contact de la zone équatoriale du sac capsulaire, cet anneau comportant une alternance de portions en forme d'arc de cercle sensiblement rigide dont les extrémités sont reliées entre elles par des zones de raccordement déformables de telle façon que la réduction de diamètre de l'anneau se fasse de façon localisée dans les éléments de raccordement flexibles.

## Revendications

1. Implant intraoculaire (10) souple destiné à être placé dans un sac capsulaire, comprenant une partie optique (12) de forme sensiblement circulaire et présentant un axe optique (xx') et une partie haptique (14) raccordée à la périphérie de la partie optique, ladite partie haptique (14) comprenant :
- une partie de contact (16) constituée par n éléments de contact (n≥ 2) (18) en forme d'arcs de cercle ayant tous le même rayon de courbure et dont le bord externe (20) est destiné à être au contact de la zone équatoriale du sac capsulaire,
- n bras de raccordement déformables (24) ayant chacun une première extrémité raccordée à la périphérie (12a) de la partie optique et une deuxième extrémité,
- n éléments de raccordement (22), chaque élément de raccordement étant raccordé à une extrémité de deux éléments de contact consécutifs, de manière que les éléments de contact et les éléments de raccordement forment un ensemble annulaire fermé ; les éléments de raccordement ayant une forme incurvée déformable et présentant une rigidité très inférieure à celle des éléments de contact ;
**caractérisé en ce que** la deuxième extrémité de chaque bras (24) est accordée à l'élément de contact correspondant dans sa partie centrale, par quoi, sous l'effet de la diminution du diamètre du sac capsulaire, les éléments de raccordement et les bras de raccordement se déforment sans entraîner de déplacement significatif de la partie optique selon la direction de son axe optique et sans induire de prise d'angulation significative de l'axe optique de l'implant par rapport à l'axe optique de l'oeil.

2. Implant intraoculaire selon la revendication 1, **caractérisé en ce que** les bras de raccordement (24) et les éléments de raccordement (22) présentent une section droite très inférieure à la section droite des éléments de contact (18).

3. Implant intraoculaire selon l'une quelconque des revendications 1 et 2, **caractérisé en ce que** la somme des longueurs des éléments de contact (18) est inférieure au périmètre du sac capsulaire dans son état contracté.

4. Implant intraoculaire selon l'une quelconque des revendications 1 à 3, **caractérisé en ce que** chaque élément de contact (18) comporte un bord (20) sensiblement parallèle à l'axe optique (xx') et deux faces respectivement antérieure (26) et postérieure (28), le bord faisant avec au moins une desdites faces un angle inférieur à 120 degrés formant ainsi une arête ou « bord carré » (30) apte à exercer une pression localisée importante sur ledit sac capsulaire.

5. Implant intraoculaire selon l'une quelconque des revendications 1 à 4, **caractérisé en ce que**, selon la direction de l'axe optique (xx'), au repos, les éléments de contact (18) sont en avant de la partie optique (12).

6. Implant intraoculaire selon l'une quelconque des revendications 1 à 5, **caractérisé en ce que**, en projection dans un plan orthogonal à l'axe optique, chaque bras de raccordement (24) est incliné par rapport à une demi-droite (0,D) portant un rayon de la partie optique.

7. Implant intraoculaire selon l'une quelconque des revendications 1 à 6, **caractérisé en ce que** l'épaisseur (e) de chaque bras de raccordement (24) selon la direction de l'axe optique est supérieure à sa largeur (ℓ) selon une direction orthogonale à l'axe optique.

8. Implant intraoculaire selon l'une quelconque des revendications 1 à 7, **caractérisé en ce que** les éléments de contact (18) présentent une angulation (b) par rapport au plan optique de la partie optique, dirigée vers le dioptre postérieur.

## Patentansprüche

1. Flexibles Intraokularimplantat (10) zum Einsetzen in einen Kapselsack, das ein im Wesentlichen kreisförmiges optisches Teil (12) mit einer optischen Achse (xx') und ein mit dem Umfang des optischen Teils verbundenes haptisches Teil (14) umfasst, wobei das haptische Teil (14) umfasst:
- ein Kontaktteil (16), bestehend aus n kreisbogenförmigen Kontaktelementen (n≥ 2) (18), die alle denselben Krümmungsradius aufweisen und deren äußerer Rand (20) für den Kontakt mit der Äquatorzone des Kapselsacks vorgesehen ist,
- n verformbare Verbindungsarme (24), die jeweils ein mit dem Umfang (12a) des optischen Teil verbundenes erstes Ende und ein zweites Ende aufweisen;
- n Verbindungselemente (22), wobei jedes Verbindungselement so mit einem Ende der beiden aufeinanderfolgenden Kontaktelemente verbunden ist, dass die Kontaktelemente und die Verbindungselemente eine geschlossene ringförmige Einheit bilden; wobei die Verbindungselemente eine verformbare Bogenform und eine sehr viel geringere Steifigkeit aufweisen als die Kontaktelemente;
**dadurch gekennzeichnet, dass** das zweite Ende jedes Arms (24) mit dem entsprechenden Kontaktelement im mittleren Teil verbunden ist, wodurch sich durch die Wirkung der Verringerung des Kapselsackdurchmessers die Verbindungselemente und die Verbindungsarme verformen, ohne eine wesentliche Verlagerung des optischen Teils in Richtung seiner optischen Achse zu bewirken und ohne eine wesentliche Winkelung der optischen Achse des Implantats in Bezug auf die optische Achse des Auges zu veranlassen.

2. Intraokularimplantat nach Anspruch 1, **dadurch gekennzeichnet, dass** die Verbindungsarme (24) und die Verbindungselemente (22) einen sehr viel geringeren Querschnitt aufweisen als der gerade Querschnitt der Kontaktelemente (18).

3. Intraokularimplantat nach einem der Ansprüche 1 und 2, **dadurch gekennzeichnet, dass** die Summe der Längen der Kontaktelemente (18) kleiner ist als der Umfang des Kapselsacks in kontraktiertem Zustand.

4. Intraokularimplantat nach einem der Ansprüche 1 bis 3, **dadurch gekennzeichnet, dass** jedes Kontaktelement (18) einen im Wesentlichen parallel zur optischen Achse (xx') verlaufenden Rand (20) und eine vordere Fläche (26) sowie eine hintere Fläche (28) aufweist, wobei der Rand mit mindestens einer der Flächen einen kleineren Winkel als 120 Grad und somit einen Grat bzw. "eckigen Rand" (30) bildet, der einen erheblichen örtlichen Druck auf den Kapselsack ausübt.

5. Intraokularimplantat nach einem der Ansprüche 1 bis 4, **dadurch gekennzeichnet, dass** die Kontaktelemente (18) in Ruhestellung in Richtung der optischen Achse (xx') vor dem optischen Teil (12) liegen.

6. Intraokularimplantat nach einem der Ansprüche 1 bis 5, **dadurch gekennzeichnet, dass** jeder Verbindungsarm (24) in Projektion in einer zur optischen Achse orthogonalen Ebene in Bezug auf eine Halbgerade (0, D) geneigt ist und einen Radius des optischen Teils trägt.

7. Intraokularimplantat nach einem der Ansprüche 1 bis 6, **dadurch gekennzeichnet, dass** die Dicke (e) jedes Verbindungsarms (24) in Richtung der optischen Achse größer als seine Breite (ℓ) in einer zur optischen Achse orthogonalen Richtung ist.

8. Intraokularimplantat nach einem der Ansprüche 1 bis 7, **dadurch gekennzeichnet, dass** die Kontaktelemente (18) eine Winkelung (b) in Bezug auf die optische Ebene des optischen Teils aufweisen, die in Richtung der hinteren Brechungsfläche gerichtet ist.

## Claims

1. A flexible intraocular implant (10) intended to be placed in a capsular bag, the implant comprising an optical portion (12) of substantially circular shape and presenting an optical axis (xx'), and a haptic portion (14) connected to the periphery of the optical portion, wherein said haptic portion (14) comprises:
- a contact portion (16) constituted by n (n≥2) contact elements (18) in the form of circular arcs all having the same radius of curvature and which an outer edge (20) is intended to be in contact with the equatorial zone of the capsular bag,
- n deformable connection arms (24), having each a first end connected to the periphery (12a) of the optical portion and a second end,
- n connection elements (22), each connection element being connected to one end of two consecutive contact elements, so that the contact elements and the connection arms constitute a closed ring assembly; the connection elements presenting a deformable curved shape, and presenting stiffness that is much less than that of the contact elements; **characterized in that** the second end of each arm is connected to the corresponding contact element in its center part whereby, under the effect of the diameter of the capsular bag diminishing, the connection elements and the connection arms deform without giving rise to significant movement of the optical portion in the direction of its optical axis, and without causing the optical axis of the implant to become significantly tilted relative to the optical axis of the eye.

2. An intraocular implant according to claim 1, **characterized in that** the connection arms (24) and the connection elements (22) present a right section that is much smaller than the right section of the contact elements (18).

3. An intraocular implant according to any of claims 1 and 2 **characterized in that** the sum of the lengths of the contact elements (18) is less than the perimeter of the capsular bag in its contracted state.

4. An intraocular according to any of claims 1 to 3 **characterized in that** each contact element (18) comprises a edge (20) substantially parallel to the optical axis (xx') and two faces, respectively, a front face (26) and a rear face (28), the edge co-operating with at least one of said two faces to form an angle of less than 120 degrees, thereby forming a square edge suitable for exerting large localized pressure on said capsular bag.

5. An intraocular implant according to any of claims 1 to 4 **characterized in that**, in the direction of the optical axis (xx'), at rest, the contact elements (18) are ahead of the optical portion (12).

6. An intraocular implant according to any of claims 1 to 5, **characterized in that**, in projection onto a plane orthogonal to the optical axis, each connection arm (24) slopes relative to a half line (0, D) carrying a radius of the optical portion.

7. An intraocular implant according to any of claims 1 to 6, **characterized in that** the thickness (e) of each connection arm (24) according to the direction of the optical axis, is greater than its width (1) in a direction orthogonal to the optical axis.

8. An intraocular implant according to any of claims 1 to 7, **characterized in that** the contact elements (18) present an angle (b) relative to the optical plane of the optical portion, each sloping towards the rear refracting surface.
